# EUROPEAN PATENT APPLICATION

(11) **EP 4 349 976 A1**
(43) Date of publication of application: **10.04.2024**
(21) Application number: 22811031.8
(22) Date of filing: 31.03.2022
(51) Int. Cl.: C12N 7/01, C12N 5/10, C12N 15/33, C12Q 1/02, G01N 33/53

(54) **METHOD FOR EVALUATING ANTIBODY-DEPENDENT ENHANCEMENT REACTION USING PSEUDOVIRUS**

(30) Priority: 27.05.2021 JP 2021089309
(71) Applicant: Mican Technologies Inc., Kyoto-shi, Kyoto 615-8245 (JP); Japan as Represented by The Director-General of National Institute of Infectious Diseases, Tokyo 162-8640 (JP); OSAKA UNIVERSITY, Suita-shi Osaka 565-0871 (JP)
(72) Inventor: SUZUKI, Ryosuke, Tokyo 162-8640 (JP); MATSUDA, Mami, Tokyo 162-8640 (JP); MURAMATSU, Masamichi, Tokyo 162-8640 (JP); SAITO, Kyoko, Tokyo 162-8640 (JP); FUKASAWA, Masayoshi, Tokyo 162-8640 (JP); HANADA, Kentaro, Tokyo 162-8640 (JP); YAMANAKA, Atsushi, Suita-shi, Osaka 565-0871 (JP); MIYAZAKI, Kazuo, Kyoto-shi, Kyoto 615-8245 (JP); YAMADA, Misuzu, Kyoto-shi, Kyoto 615-8245 (JP); SHIMIZU, Jun, Kyoto-shi, Kyoto 615-8245 (JP)
(74) Representative: J A Kemp LLP
(86) International application number: PCT/JP2022/016731
(87) International publication number: WO 2022/249757

(57) **Abstract**

The present invention aims to provide a method for testing the function of antibodies that uses safe antigens and gives results more quickly. The present invention relates to a method for determining antibody-dependent enhancement ability of antibodies, including contacting, in the presence of a test antibody, Fcγ receptor-expressing cell with single round infectious virus particles containing a gene with a region encoding a labeled protein and a region encoding non-structural (NS) proteins 1 to 5 of the yellow fever virus genome, a capsid protein of a virus, and an outer shell protein (Envelope) of a virus, wherein when the measured label is greater than that of a negative control cell, the test antibody is determined to have an antibody-dependent enhancement ability, and the like.

## Description

### [Technical Field]

The present invention relates to a method for functional evaluation of antibody, which uses pseudovirus.

### [Background Art]

Dengue virus, which belongs to the genus Flavivirus of the Flaviviridae family, is a +strand RNA virus, and its genome consists of three structural protein regions (C, prM, E) and seven non-structural protein regions (NS1, NS2a, NS2b, NS3, NS4a, NS4b, NS5). Dengue virus includes four types of viruses (DENV-1 to DENV-4) and they are biologically classified as serotypes. Dengue virus is transmitted by mosquitoes. When a dengue virus-carrying mosquito bites a human, the injected virus first infects dendritic cells, monocytes, and lymphocytes in the skin and undergoes primary proliferation. The virus then travels through the bloodstream to various organs in the body and undergoes secondary proliferation. The condition in which the proliferated virus is present in the blood is called viremia, and when a mosquito sucks such blood, it becomes a new virus-carrying mosquito and can transmit the virus to other humans.

Dengue virus becomes epidemic mainly in tropical/subtropical regions where the main mosquito vector, Aedes aegypti, lives. In recent years, it is known that the Aedes albopictus, which lives in temperate regions including Japan, also transmits dengue virus. When a domestic dengue virus epidemic occurred mainly in the Kanto region in 2014, the Aedes albopictus was the vector animal. The population at risk of being infected with dengue virus is estimated to be 3.9 billion people, and 390 million people, which is one-tenth the number, are infected with some kind of dengue virus every year. Even when humans are infected with the dengue virus, many of the infections are inapparent infections, and only about 100 million of them actually develop symptoms.

As described above, even though many infected people show inapparent infection, some of the infected people may develop dengue fever, a transient febrile disease. The main symptoms of dengue fever are relatively mild, including fever, headache, muscular pain, and joint pain. Most cases of dengue fever are mild, but a part of patients with dengue fever develop a severe form, dengue hemorrhagic fever. The symptoms of dengue hemorrhagic fever (plasma leakage, bleeding tendency) are severe and can lead to death if proper treatment is not provided. It has been epidemiologically proven that first infection often causes dengue fever and the second infection with a dengue virus of a serotype different from the first infection increases the risk of developing dengue hemorrhagic fever. It is said that a third or subsequent infection does not occur.

Although the mechanism of severe progression in dengue viral infection has not been completely elucidated, it is known that higher levels of viremia tend to result in severe progression. Antibody-dependent enhancement (ADE) is one of the leading theories for the onset mechanism of severe progression, and induces hyperviremia. ADE is a phenomenon in which antibodies, which are supposed to neutralize viruses, actually worsen the viral infection. Antibodies that have bound to virus promote viral invasion into cells via Fcγ receptor on the cell surface and increase infection.

It has been reported that the number of severe cases increased in the vaccinated group because antibodies induced by inoculation with dengue vaccine (Dengvaxia) showed ADE. In particular, when this vaccine was inoculated to people who were negative for dengue antibodies, they experienced a pseudo-initial infection and the risk of severe progression at next infection increased. For this reason, only those who are positive for dengue antibodies can be vaccinated with Dengvaxia at present. Previous studies from overseas have shown epidemiological reports stating that ADE confirmed in vitro is correlated with severe progression in vivo.

Simple rapid kits (immunochromatography, ELISA method) are commercially available for antibody test in infectious diseases. They are all qualitative and quantitative chemical test methods and do not reveal biological functional activities of antibody. The outline of the ADE test is to examine how much infection of cells can be increased by adding antibodies in the presence of the minimum necessary amount of virus. A method for evaluating ADE using fixed cells has been reported (Non Patent Literature 1). Existing ADE tests can examine biological functions of antibodies, but require live dengue virus as the assay antigen. Dengue virus is classified as a Category IV infectious disease under the Infectious Disease Control Act. Therefore, biosafety level 2 (BSL2) equipment is required when handling Dengue virus. Furthermore, since this method requires at least three days to obtain results, it is realistically difficult to obtain results before patients hospitalized with dengue fever become seriously ill.

Single Round Infectious Particles (SRIPs) are one time infectious particles. SRIPs that retain the outer shell of a specific virus can be produced by using the outer shell gene of the virus when producing SRIPs (Non Patent Literatures 1 to 4). SRIP contains in its inside a self-replicating gene called replicon. Although replicon genes are replicated within SRIPs-infected cells, they cannot be amplified as particles (viruses) and can be safely handled because they do not cause cell destruction or reinfection. By encoding nanoluciferase as a reporter gene, infected cells can be measured based on luciferase activity. Since SRIPs bind to antibodies as pseudoviruses, they can be used as antigens for tests of antibody functions such as antibody neutralization activity and ADE activity. ADE activity measurement using SRIPs and the luciferase activity produced thereby has also been reported (Non Patent Literature 4), but this method also required three days for measurement.

Other functional antibody tests include neutralization tests. In the neutralization test, an antibody is added to a site where a large amount of antigen (virus) is present and how much virus can be reduced is determined. Neutralization tests also previously required a live virus as the assay antigen. A neutralization test using SRIPs as substitute antigens and detecting the luciferase activity produced by SRIPs that infected cells has been reported (Non Patent Literatures 5 to 6). However, it requires 3 days of culture since it takes time for the replicon to increase within the cells after the reaction with the neutralizing antibody.

### [Citation List]

### [Non Patent Literature]

[NPL 1]
   Eiji Konishi et al., Journal of Virological Methods; 163:360-367 (2010)
[NPL 2]
   Ryosuke Suzuki et al., Journal of General Virology; 95:60-65 (2014)
[NPL 3]
   Atsushi Yamanaka et al., Vaccine; 32(34) :4289-95(2014)
[NPL 4]
   Atsushi Yamanaka et al., Microbes and Infection; 18:277-284 (2016)
[NPL 5]
   Atsushi Yamanaka et al., Journal of General Virology:DOI 10.1099/jgv0.000669(2017)
[NPL 6]
   Mami Matsuda et al., Scientific Repors; DOI 10.1038/s41598-018-34865-y(2018)

### [Summary of Invention]

### [Technical Problem]

Therefore, in order to generalize neutralization tests and ADE tests, there was a demand for a method in which safe antigens can be used and by which the results can be obtained more quickly.

### [Solution to Problem]

The present inventors have conducted intensive studies of Single Round Infectious Particles (SRIPs) suitable as assay antigens for neutralization tests and ADE tests. As a result, they have found that the use of yellow fever virus (YFV) gene as the backbone of the replicon plasmid results in rapid uptake of SRIPs into cells and accelerated replication thereof, thereby shortening the time up to the detection, and successfully developed a rapid diagnostic method for antibody function.

That is, the present invention relates to the following.

[1] A single round infectious virus particle comprising a gene comprising a region encoding a labeled protein and a region encoding non-structural (NS) proteins 1 to 5 of the yellow fever virus genome,
   a capsid protein of a virus, and
   an outer shell protein (Envelope) of an evaluation target virus.
[2] The pseudo-virus particle of [1], wherein the gene encoding the aforementioned labeled protein (nanoluciferase) can be expressed only in cells infected with the aforementioned single round infectious virus particle.
[3] The virus particle of [1] or [2], wherein the aforementioned labeled protein is nanoluciferase.
[4] The virus particle of any one of [1] to [3], wherein the outer shell protein of the evaluation target virus is an outer shell protein of dengue virus.
[5] The virus particle of any one of [1] to [4], wherein a CMV promoter is linked upstream of the aforementioned region encoding a labeled protein and the region encoding non-structural (NS) proteins 1 to 5 of the yellow fever virus genome, so that the gene expression is directly controlled by the CMV promoter.
[6] A method for producing the single round infectious virus particle of any one of [1] to [5], comprising infecting animal cells with a vector comprising a gene with a region encoding non-structural (NS) proteins 1 to 5 of the yellow fever virus genome and a region encoding a labeled protein, a vector comprising a gene encoding a capsid protein of a virus gene, and a vector comprising a gene encoding an outer shell protein of an evaluation target virus, and collecting the single round infectious virus particle of any one of [1] to [5] produced within the infected cells.
[7] The production method of [6], wherein the aforementioned gene encoding the capsid protein of the virus, and/or the gene encoding the outer shell protein of the evaluation target virus are/is linked to a CAG promoter so that the gene(s) are/is directly controlled by the CAG promoter.
[8] A method for determining neutralization activity of an antibody, comprising
   contacting an Fcγ receptor-non-expressing cell with the single round infectious virus particle of any one of [1] to [5] in the presence of a test antibody,
   culturing the aforementioned cells for 24 to 48 hr, and
   measuring a label in a culture medium resulting from the aforementioned culture, wherein
   when the level of measured label is lower than that measured in a negative control cell, the test antibody is determined to have neutralization activity on the aforementioned evaluation target virus.
[9] The method of [8], wherein the aforementioned Fcγ receptor-non-expressing cell is Vero cell.
[10] A method for determining antibody-dependent enhancement ability of an antibody, comprising
   contacting an Fcγ receptor-expressing cell with the single round infectious virus particle of any one of [1] to [5] in the presence of a test antibody,
   culturing the aforementioned cells for 30 min to 24 hr, and
   measuring a label in the aforementioned culture medium, wherein
   when the level of the aforementioned measured label is higher than that measured in a negative control cell, the test antibody is determined to have antibody-dependent enhancement ability for the aforementioned evaluation target virus.
[11] The method of [10], wherein the aforementioned Fcγ receptor-expressing cell is K562 cell or Mylc cell.
[12] The method of [10] or [11], wherein the aforementioned Fcγ receptor-expressing cell is cultured for 2 to 16 hr.
[13] The method of any one of [10] to [12], wherein a dilution rate of the single round infectious virus particle is 2 times or more.
[14] The method of any one of [10] to [13], wherein the culture of the Fcγ receptor-expressing cell is performed using Fcγ receptor-expressing cells at not less than 2.1×10² cells/well in a 96 well plate.
[15] The method of any one of [10] to [14], comprising adding a composition comprising the test antibody to the culture medium before contact of the aforementioned single round infectious virus particle, or simultaneously with the contact of the aforementioned single round infectious virus particle.
[16] The method of [15], wherein a mixture of the aforementioned single round infectious virus particle and the composition comprising the test antibody is added to the culture medium.
[17] The method of [15] or [16], wherein the aforementioned composition comprising the test antibody is a serum derived from a test subject.
[18] The method of [17], wherein the aforementioned composition comprising the test antibody is a 10- to 10240-fold diluted serum derived from the test subject.
[19] A composition for determining antibody function comprising the single round infectious virus particle of any one of [1] to [5] as an active ingredient.
[20] The composition of [19], the antibody function is antibody-dependent enhancement ability or neutralization activity.
[21] The composition of [19] or [20] for use in the method of any one of [6] to [18].
[22] A kit for determining antibody function comprising the single round infectious virus particle of any one of [1] to [5] as an active ingredient.
[23] The kit of [22], further comprising a negative control antibody or a standard antibody.
[24] The kit of [22] or [23], further comprising an Fcγ receptor-expressing cell.

### [Advantageous Effects of Invention]

Using the SRIPs having YFV replicon of the present invention, antibody function can be determined in a shorter period of time (1 to 2 days) than conventional (3 days), whereby rapid antibody tests can be performed. This allows for rapid determination of the possibility that test subjects develop ADE due to reinfection, as well as its use for property evaluation of antibodies (neutralization activity evaluation) in the development of pharmaceutical products and evaluation of induction potency of ADE active antibodies of vaccine.

### [Brief Description of Drawings]

[Fig. 1]

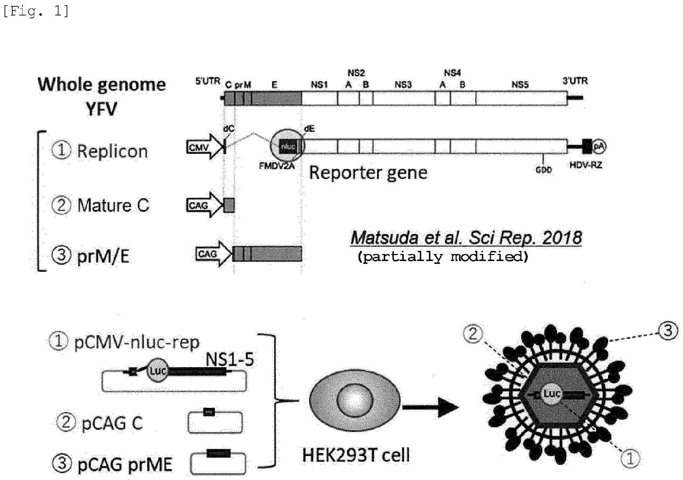

   Fig. 1 is a diagram schematically showing the information on the three types of plasmids necessary to produce SRIP (partially modified from Matsuda et al., Sci Rep. 2018), and the production method and constituent components of SRIP.
[Fig. 2]

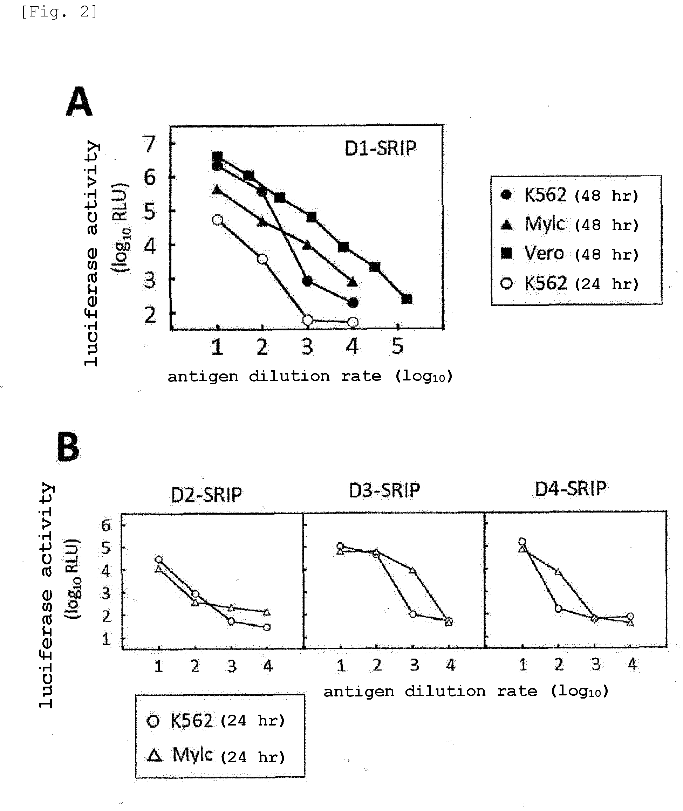

   Fig. 2 shows diagrams showing the measurement results of luciferase activity in cultured cells (K562, Mylc, or Vero cells) 24 to 48 hr after infecting the cells with dengue virus SRIP in an antigen concentration-dependent manner, in which A shows the detailed results for dengue type 1 virus SRIP (D1-SRIP), and B shows the results for dengue types 2 to 4 virus SRIPs (D2-SRIP to D4-SRIP).
[Fig. 3]

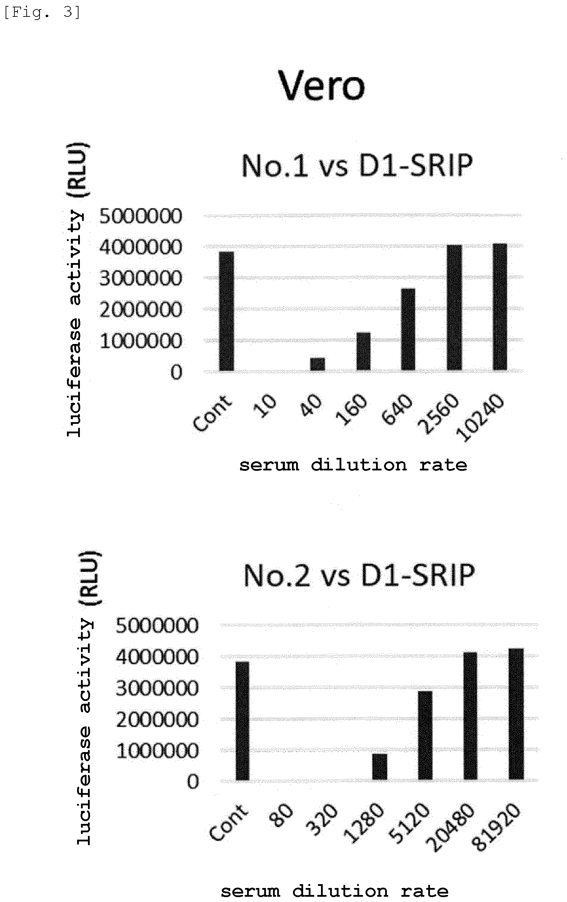

   Fig. 3 shows diagrams showing the measurement results of luciferase activity after 48 hr using Vero cells, as the neutralization activity of dengue antibody-positive human serum against D1-SRIP.
[Fig. 4]

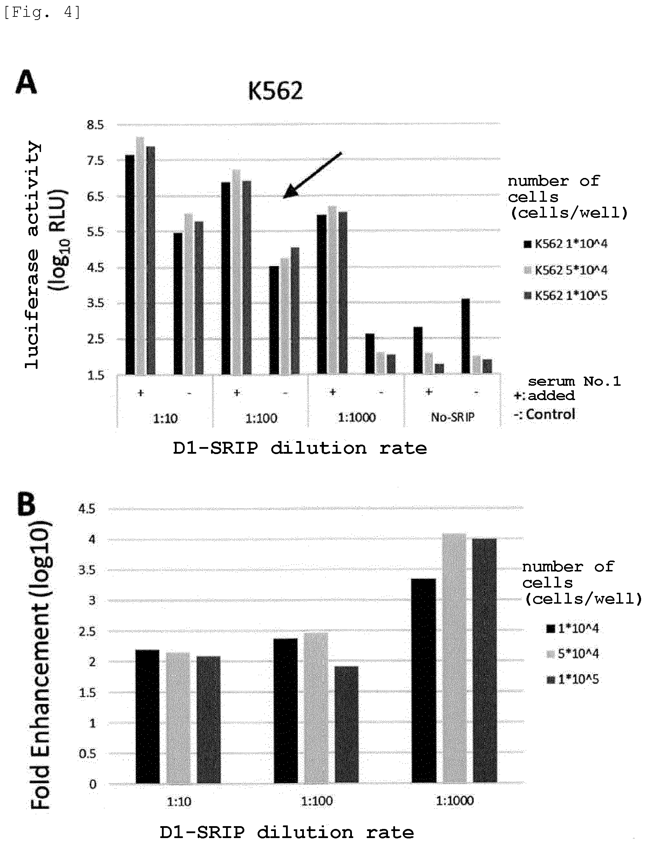

   Fig. 4 shows diagrams showing (A) the measurement results of luciferase activity after 24 hr using K562 cells and (B) the results converted to Fold Enhancement, as the enhancement activity of dengue antibody-positive human serum against D1-SRIP.
[Fig. 5]

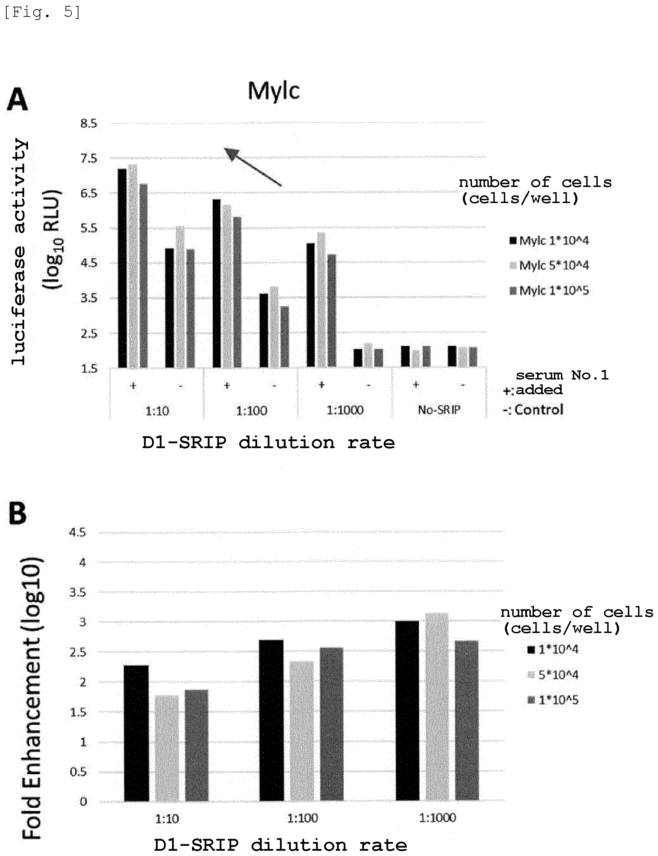

   Fig. 5 shows diagrams showing (A) the measurement results of luciferase activity after 24 hr using Mylc cells and (B) the results converted to Fold Enhancement, as the enhancement activity of dengue antibody-positive human serum against D1-SRIP.
[Fig. 6]

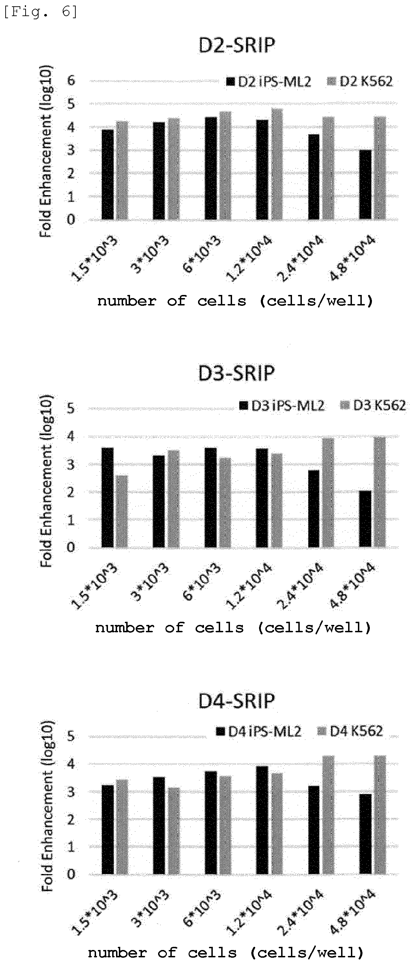

   Fig. 6 shows diagrams showing the Fold Enhancement results converted from luciferase activity measured after 24 hr using K562 and Mylc cells, as the enhancement activity of dengue antibody-positive human serum against D2-SRIP to D4-SRIP.
[Fig. 7]

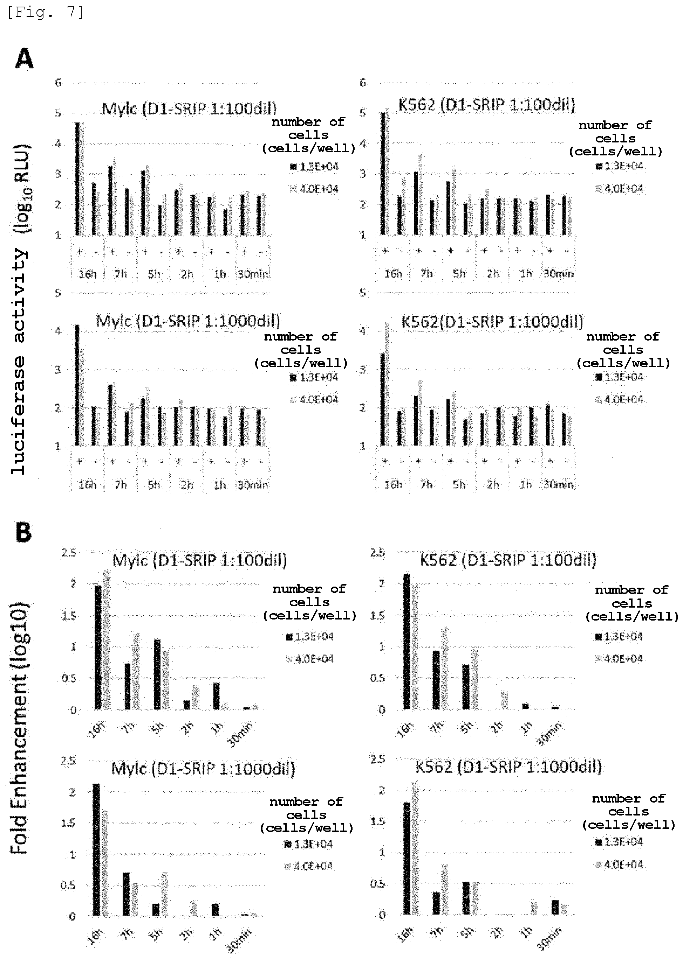

   Fig. 7 shows diagrams showing (A) the measurement results of luciferase activity 30 min to 16 hr after infection and using K562 and Mylc cells and (B) the results converted to Fold Enhancement, as the enhancement activity of dengue antibody-positive human serum against D1-SRIP.
[Fig. 8]

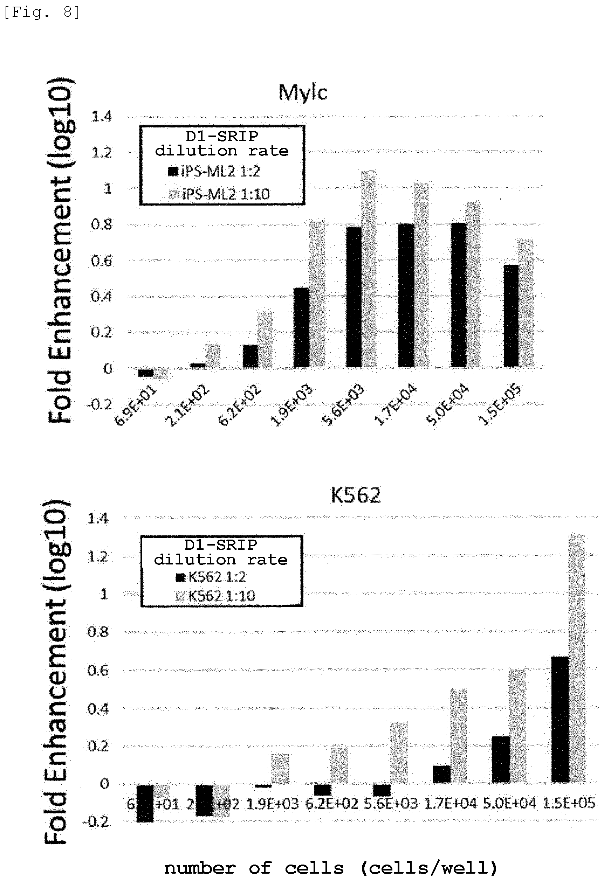

   Fig. 8 shows diagrams showing the Fold Enhancement results converted from luciferase activity measured after 5 hr using high concentration SRIP antigen and low to high density K562/Mylc cells, as the enhancement activity of dengue antibody-positive human serum against D1-SRIP.
[Fig. 9]

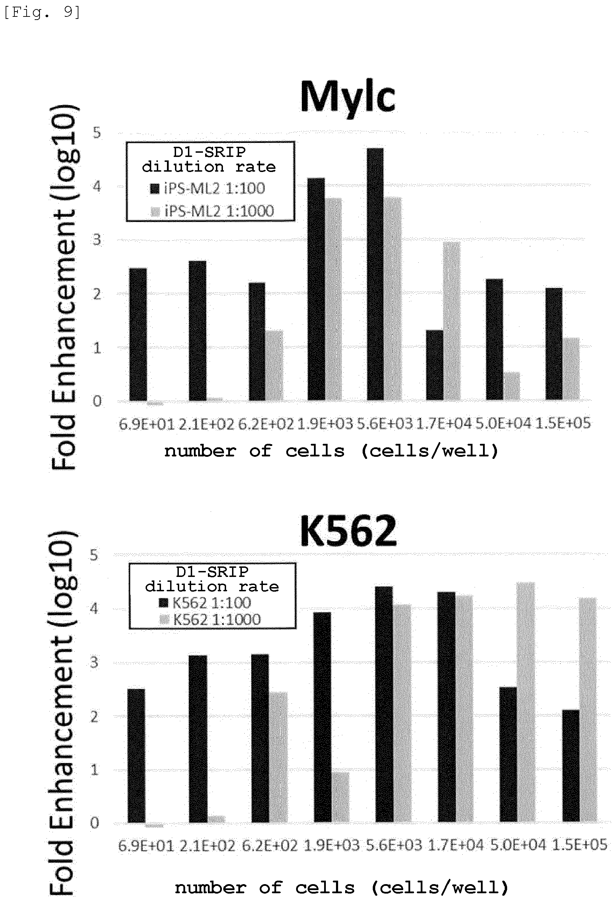

   Fig. 9 shows diagrams showing the Fold Enhancement results converted from luciferase activity measured after 48 hr using low concentration SRIP antigen and low to high density K562/Mylc cells, as the enhancement activity of dengue antibody-positive human serum against D1-SRIP.
[Fig. 10]

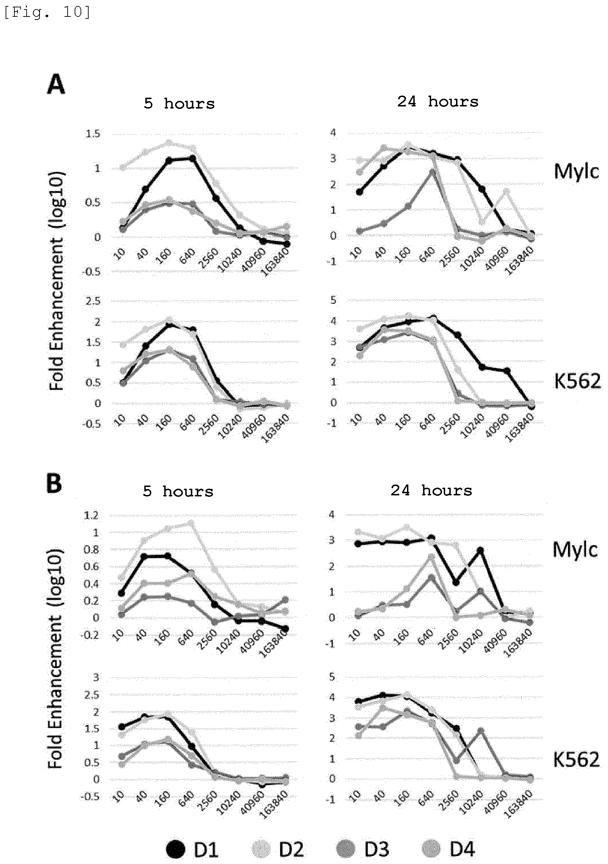

   Fig. 10 shows diagrams showing the results of concentration-dependent enhancement activity of dengue antibody-positive human serum against D1-SRIP to D4-SRIP, obtained by performing an enhancement test under optimized conditions.

### [Description of Embodiments]

### (Single round infectious virus particles (SRIPs))

The present invention relates to single round infectious virus particles (SRIPs) containing a gene having a region encoding a labeled protein and a region encoding non-structural (NS) proteins 1 to 5 of the yellow fever virus genome, a capsid protein of a virus, and an outer shell protein (Envelope) of an evaluation target virus.

SRIPs in the present specification have a region encoding a labeled protein and a region encoding non-structural (NS) proteins 1 to 5 of the yellow fever virus genome in the gene thereof. The "labeled protein" is not particularly limited as long as it is a protein capable of labeling cells infected with the aforementioned SRIPs. Preferably, it can be expressed only in cells infected with the aforementioned SRIPs. The labeled protein is preferably a fluorescent protein or a luminescent protein. Specifically, green fluorescent protein (GFP), red fluorescent protein (RFP), blue fluorescent protein (BFP), cyan fluorescent protein (CFP), yellow fluorescent protein (YFP), fluorescent proteins improved from these fluorescent proteins (see https://www.fpbase.org/), luciferase, and nanoluciferase can be mentioned.

Yellow fever virus is a virus that belongs to the genus Flavivirus of the Flaviviridae family, and its genome is a single-stranded plus RNA. In the present specification, therefore, the "gene" may be DNA or RNA. In the virus particles of the genus Flavivirus, a nucleocapsid in which a capsid protein (also called core protein) is bound to a single-stranded RNA is surrounded by membrane protein and envelope proteins. One reading frame is present on single-stranded RNA, and three types of structural proteins (C, prM, E) and seven types of non-structural proteins (NS1, NS2A, NS2B, NS3, NS4A, NS4B, NS5) are encoded. The "non-structural (NS) proteins 1 to 5 of the yellow fever virus genome" means these seven types of non-structural proteins. Proteins with mutations in a part (e.g., 1 to 5 amino acids) of these proteins are also included in the non-structural (NS) proteins 1 to 5 of the yellow fever virus genome as long as they are considered to be substantially the same as these proteins and the effects of the present invention are not affected.

The gene of SRIPs in the present specification optionally has, where necessary, a region other than a region encoding the aforementioned labeled protein and a region encoding non-structural (NS) proteins 1 to 5 of the yellow fever virus genome. However, in order to acquire the function of one time infectivity, it does not contain gene regions encoding at least (i) a capsid protein, and (ii) one or both of a membrane protein and an envelope protein. That is, since the virus particles in the present specification have the Envelope of the evaluation target virus, they can infect cells that are infected by the evaluation target virus, whereas they cannot proliferate within the infected cells because the gene thereof does not encode capsid protein, and/or Envelope and membrane protein precursor.

For example, a region encoding a labeled protein and a region encoding non-structural (NS) proteins 1 to 5 of the yellow fever virus genome may be linked to transcriptional regulatory regions that can be expressed in cells used in infection evaluation tests. Such transcription regulatory region optionally contains a promoter. The promoter is not particularly limited as long as it has activity in the aforementioned cells. For example, it may be a constitutive promoter, and specifically, cytomegalovirus (CMV) promoter, Rous sarcoma virus (RSV) promoter, CAG promoter (promoter having cytomegalovirus enhancer, chicken β-actin promoter, and rabbit β-globin gene splicing acceptor), ubiquitin promoter, Simian virus 40 (SV40) promoter, and elongation factor 1α (EF-1α) promoter can be mentioned. A promoter is linked upstream of the aforementioned protein-encoding region so as to directly control the expression of the region.

The "capsid protein of a virus" that the SRIPs in the present specification has is a protein that binds to the aforementioned RNA gene to form a nucleocapsid. The virus from which the capsid protein is derived is not particularly limited as long as it belongs to the Flaviviridae family. Examples thereof include yellow fever virus (YFV), dengue virus (DENV), Japanese encephalitis virus (JEV), West Nile virus (WNV), and tick-borne encephalitis virus (TBEV), and YFV and DENV are preferred.

The outer shell protein (Envelope) is a protein that constitutes the outer shell of virus, and has the functions of binding to receptors and membrane fusion. The evaluation target virus from which the Envelope is derived is not particularly limited as long as it belongs to the aforementioned Flavivirus genus, and may be, for example, DENV. DENV includes four kinds of type 1 to type 4, which are called DENV-1, DENV-2, DENV-3, and DENV-4. Throughout the present specification, DENV may be any one of DENV-1 to DENV-4, a plurality of them, or all of them.

SRIPs in the present specification may further contain a membrane protein in addition to those mentioned above.

SRIPs in the present specification can be obtained by introducing a replicon vector containing a gene having a region encoding a labeled protein and a region encoding non-structural (NS) proteins 1 to 5 of the yellow fever virus genome, a vector containing a gene encoding a capsid protein, and a vector containing a gene encoding Envelope and a gene encoding a membrane protein precursor (prM) into the same cell (Fig. 1). Therefore, the present invention relates to a method for producing SRIPs, including introducing a vector containing a gene having a region encoding non-structural (NS) proteins 1 to 5 of the yellow fever virus genome and a region encoding a labeled protein, a vector containing a gene encoding a capsid protein of a virus, and a vector containing a gene encoding an outer shell protein of an evaluation target virus into animal cells, and collecting SRIPs produced in the introduced cells. The vector may be preferably a plasmid vector. The animal cells may be human cells or monkey cells and, for example, HEK293T cells can be used. Each viral gene is known in the technical field and can be obtained from various databases.

### (Neutralization activity evaluation)

The neutralization activity of antibody can be measured in a short time by infecting Fcγ receptor-non-expressing cells with the aforementioned SRIPs in the presence of a test antibody. Therefore, the present invention provides a method for determining neutralization activity of an antibody, including contacting Fcγ receptor-non-expressing cells with the aforementioned SRIPs in the presence of a test antibody, culturing the aforementioned cells for 24 to 72 hr, and measuring a label in a culture medium resulting from the aforementioned culture, wherein when the measured label is less than the label measured in negative control cells similarly contacted with SRIPs in the absence of the antibody or in the presence of a negative control antibody, the test antibody is determined to have neutralization activity on the aforementioned evaluation target virus.

The Fcγ receptor-non-expressing cell may be any cell that does not express Fcγ receptors, and is preferably an epithelial cell, such as human or primate (monkey, chimpanzee, etc.) cell. A specific example of the Fcγ receptor-non-expressing cell is Vero cell.

The aforementioned SRIPs may be brought into contact with Fcγ receptor-non-expressing cells in the presence of a test antibody by adding the aforementioned SRIPs to the culture medium of the Fcγ receptor-non-expressing cells after or simultaneously with the addition of the test antibody to the culture medium, or by adding the test antibody and the aforementioned SRIPs mixed in advance to the culture medium of the Fcγ receptor-non-expressing cells. For example, a test antibody and SRIPs are mixed in advance, and the mixture is allowed to stand at about 37°C for 1 to 6 hr (preferably, 2 hr) and then contacted with Fcγ receptor-non-expressing cells. As the test antibody, an antibody composition as well as a purified antibody can be used. For example, serum derived from a test subject can be used as is or used after dilution. For dilution, it can be diluted 5120-fold or less, 640-fold or less, 320-fold or less, 160-fold or less, 80-fold or less, 40-fold or less, or 10-fold or less.

The time of contact of SRIPs in the presence of a test antibody is not less than 24 hr and less than 72 hr, preferably 24 to 48 hr.

The label can be measured as appropriate according to the kind of the selected label. For example, luminescence can be measured using a luminometer.

Neutralization activity is determined by comparing the measured level of label with the level of label in the negative control. A negative control is an Fcγ receptor-non-expressing cell infected with SRIPs under the same conditions as the test for the test antibody except that neutralization activity is not present. Specifically, the negative control may be an Fcγ receptor-non-expressing cell infected with SRIPs in the absence of a test antibody, or an Fcγ receptor-non-expressing cell infected with SRIPs in the presence of an antibody that evidently does not have neutralization activity (e.g., antibodies that do not bind to the evaluation target virus). The level of the label of the negative control can also be obtained by performing the above-mentioned neutralization activity evaluation method simultaneously with the test antibody, or may be obtained by performing the above-mentioned neutralization activity evaluation method separately from the test antibody. When the level of the label is low as compared with the negative control, the test antibody can be determined to have the neutralization activity. For example, when patient serum is used as the test antibody and the level of the label in the presence of the serum is lower than that of a negative control, the patient can be determined to have an antibody with neutralization activity.

Throughout the present specification, the "level" means an indicator of quantified abundance and includes, for example, concentration, amount, or an indicator that can be used in place of these. Therefore, the level may be a measured value itself such as fluorescence intensity, or may be a value converted to concentration. In addition, the level may be an absolute numerical value (abundance, abundance per unit area, etc.), or may be a relative numerical value compared to a comparison control set as necessary.

### (Antibody-dependent enhancement (ADE) ability evaluation)

The antibody-dependent enhancement ability (ADE) of antibody can be measured in a short time by infecting Fcγ receptor-expressing cells with the aforementioned SRIPs in the presence of a test antibody. Therefore, the present invention provides a method for determining antibody-dependent enhancement ability of an antibody, including contacting Fcγ receptor-expressing cells with the aforementioned SRIPs in the presence of a test antibody, culturing the aforementioned cells for 30 min to 24 hr, and measuring a label in the aforementioned culture medium, wherein when the level of the aforementioned measured label is higher than that measured in a negative control cell, the test antibody is determined to have antibody-dependent enhancement ability for the aforementioned evaluation target virus.

The Fcγ receptor-expressing cell may be any cell that expresses Fcγ receptors, and is preferably a cell of the immune system, such as human or primate (monkey, chimpanzee, etc.) cell. A specific example of the Fcγ receptor-expressing cell includes Mylc cell (MiCAN Technologies, Inc., Japan) and K562 cell.

The aforementioned SRIPs may be brought into contact with Fcγ receptor-expressing cells in the presence of a test antibody by adding the aforementioned SRIPs to the culture medium of the Fcγ receptor-expressing cells after or simultaneously with the addition of the test antibody to the culture medium, or by adding the test antibody and the aforementioned SRIPs mixed in advance to the culture medium of the Fcγ receptor-expressing cells. As the test antibody, an antibody composition as well as a purified antibody can be used. As the antibody composition, for example, serum derived from a test subject can be used as is or used after dilution. For dilution, it can be diluted 10-fold or more, 40-fold or more, 160-fold or more and/or 10240-fold or less, 2560-fold or less, 640-fold or less, or may be any combination of these upper limit values and lower limit values, for example, 10-fold or more and 10240-fold or less, or 40-fold or more and 2560-fold or less. SRIPs obtained by the above-mentioned method can be diluted as appropriate and used in this method. In this case, the dilution rate can be 2-fold or more, and may be, for example, 10-fold or more, 100-fold or more, or 1000-fold or more.

Cell culture can be performed, for example, on a plate capable of cultivating immune cells, such as a 96-well plate. The number of cells to be cultured when using a 96-well plate can be set to not less than 6.9×10 cells/well, not less than 2.1×10² cells/well, not less than 6.2×10² cells/well, not less than 1.9×10³ cells/well, not less than 5.6×10³ cells/well, or not less than 1.7×10⁴ cells/well. The number of cells to be cultured can be appropriately determined according to the dilution rate of SRIPs and the cells to be used. For example, when Mylc cells are used, the number of cells on a 96-well plate is preferably set to not less than 1.9×10³ cells/well.

The time for contacting SRIPs with cells in the presence of a test antibody (cell culture time in the presence of the test antibody and SRIPs) is 30 min to 24 hr, or may also be, for example, 2 to 24 hr, 5 to 24 hr, 30 min to 16 hr, 2 to 16 hr, or 5 to 16 hr.

Label can be measured as appropriate according to the kind of the label selected. For example, luminescence can be measured using a luminometer.

ADE ability is determined by comparing the measured level of label with the level of label in the negative control. A negative control is an Fcγ receptor-expressing cell infected with SRIPs under the same conditions as the test for the test antibody except that neutralization activity is not present. Specifically, the negative control may be an Fcγ receptor-expressing cell infected with SRIPs in the absence of a test antibody, or an Fcγ receptor-expressing cell infected with SRIPs in the presence of an antibody that evidently does not have ADE ability (e.g., antibodies that do not bind to the evaluation target virus). The level of the label of the negative control can also be obtained by performing the above-mentioned ADE ability evaluation method simultaneously with the test antibody, or may be obtained by performing the above-mentioned ADE ability evaluation method separately from the test antibody. When the level of the label is high as compared with the negative control, the test antibody can be determined to have the ADE ability. For example, when patient serum is used as the test antibody and the level of the label in the presence of the serum is higher than that of a negative control, the patient can be determined to have an antibody with ADE ability.

### (Composition for determining antibody function)

As described above, the SRIPs of the present invention can be used for the measurement of the functions (neutralization activity, ADE ability) of an antibody. Therefore, the present invention relates to a composition for determining antibody function containing the aforementioned SRIPs as an active ingredient. The antibody function here is a function related to viral infection and is typically neutralization activity or antibody-dependent enhancement ability. The composition of the present invention can be a composition for use in the aforementioned neutralization activity evaluation method or ADE ability evaluation method. The composition can contain components such as stabilizer, buffer, and the like in addition to the aforementioned SRIPs.

### (Kit for determining antibody function)

Also, the present invention relates to a kit for determining antibody function, containing the aforementioned SRIPs. The antibody function here is a function related to viral infection and is typically antibody-dependent enhancement ability or neutralization activity. The kit may further contain a negative control antibody or a standard antibody, and/or an Fcγ receptor-non-expressing cell (when the antibody function is neutralization activity) or an Fcγ receptor-expressing cell (when the antibody function is ADE). The kit of the present invention may further contain an outer box, container, diluent, culture medium, and/or instructions. In the kit for determining antibody function of the present invention, different components may be packaged in separate containers and included in one kit, or only some components (including at least the aforementioned SRIPs) may be included in the kit, and other components may be provided separately from the kit.

The present invention is explained in more detail in the following using Examples, but they are not intended to limit the scope of the present invention. The documents cited throughout the present specification are incorporated in their entirety in the present specification by reference.

### (Example 1) Production of SRIPs

According to the procedures of a previous report (Matsuda, M. et al., Sci Rep; 8:16624(2018)), luciferase-expressing SRIPs were produced based on the prM/E gene information of dengue type 1 virus, dengue type 2 virus (JX042506), dengue type 3 virus (EU081222), and dengue type 4 virus (AY618988). Schematic diagram of SRIPs production process is shown in Fig. 1.

### (1) Construction of replicon plasmid

Specifically, a YFV subgenomic replicon plasmid was constructed using the 17D-204 strain of yellow fever virus (YFV) (Fig. 1, circle 1). Virus RNA was extracted from YFV-infected Vero cells, reverse transcribed into cDNA, and amplified as a separate dsDNA fragment containing cytomegalovirus (CMV) promoter, nanoluciferase (NanoLuc) gene before foot-and-mouth disease virus (FMDV) 2A gene, and hepatitis delta virus ribozyme (HDV-RZ). The NanoLuc gene was introduced at a position 21aa after the C-coding region, followed by the RMDV-2A gene and at a position 24aa before the C-terminal transmembrane domain of the E protein-coding region. Some individual fragments required for the production of replicon-length cDNA were inserted into the low copy number plasmid pACYC177 and named pCMV-YF-nluc-rep.

### (2) Construction of capsid expression plasmid

RNA was extracted from Vero cells infected with YFV and reverse transcribed into cDNA. A cDNA encoding 101aa mature capsid (C) was amplified from the obtained cDNA. The obtained fragment was cloned into pCAGGS to construct a plasmid pCAG-YF-C expressing the YFV capsid (Fig. 1, circle 2).

### (3) Construction of prME-expression plasmid

Plasmid pcD1ME^{YG1} encoding pre-membrane/membrane (prM/M) of dengue type 1 virus (DENV-1) (D1/Hu/Saitama/NIID100/2014) and envelope (E) protein was prepared by a previously reported method (Konishi et al., Vaccine; 24(12):pp.2200-2207 (2006)). Plasmids pcD2ME, pcD3ME^{SG}, pcD4ME^{Th} encoding pre-membrane/membrane (prM/M) of each of dengue type 2 virus (DENV-2) (D2/New Guinea C/1944), dengue type 3 virus (DENV-3) (D3/SG/05K4454DK1/2005), and dengue type 4 virus (DENV-4) (D4/Thailand/0476/1997), and envelope (E) proteins were prepared by a previously reported method (Yamanaka, A. et al., (2014), Matsuda, M. et al., (2018) supra) (Fig. 1, circle 3).

### (4) Production of SRIPs

293T cells were proliferated in a 10 cm dish and cotransfected with three kinds of plasmids using polyethyleneamine (Fig. 1): 2.5 µg replicon plasmid, 1.25 µg YFV capsid-expression plasmid, and 1.25 µg each DENV prME-expression plasmid. Two days after transfection, the medium was removed and replaced with a new medium containing 10 mM HEPES buffer. Three days after transfection, the medium was collected and used as SRIPs. The infection titer of purified SRIPs was estimated by luciferase assay of infected Vero cells (Fig. 2). SRIPs constituted of YFV capsid (C) and replicon plasmid and DENV-1 pre-membrane/membrane (prM/M), and envelope (E) was named D1-SRIPs. Similarly, SRIPs constituted of YFV capsid (C) and replicon plasmid and DENV-2, DENV-3 and DENV-4 pre-membrane/membrane (prM/M), and envelope (E) were respectively named D2-SRIPs, D3-SRIPs, and D4-SRIPs.

### (Example 2) SRIP infection experiment

K562 cells which are human leukemia cells, and Mylc cells which are immortalized myeloid cells (MiCAN Technologies, Inc.) were used as Fcγ receptor-expressing cells. In addition, Vero cells (Fcγ receptor-non-expressing), which are commonly used in dengue virus infection experiments, were used as Comparative Example. As a culture medium, RPMI-1640 was used for K562 cells, α-modified Eagle Minimum Essential Medium (αMEM) was used for Mylc cells, and Dulbecco's modified Eagle medium (DMEM) was used for Vero cells, and 10% fetal bovine serum (FBS) was added to each and used. 40 µL of the step-diluted D1-SRIPs were inoculated into each cell (0.25 to 1.0×10⁵ cells), allowed to adsorb for 2 hr at 37°C, and then 100 µL of warmed culture medium was dispensed into each well and further cultured at 37°C. After 24 to 48 hr, the cells were washed once with 300 µL of phosphate-buffered saline (PBS) per well and lysed with 30 µL of Passive Lysis 5X Buffer (E1941) of Promega KK. After thorough stirring, the cell lysate (10 µL) and Promega KK's luciferase substrate, Nano-Glo (registered trademark) Luciferase Assay System (N1110) (10 µL) were mixed in a white microplate, and luciferase activity was measured with a 96-well plate compatible luminometer GloMax (registered trademark) Navigator System of Promega K.K. Similarly, K562 cells and Mylc cells were infected with D2-SRIPs, D3-SRIPs, and D4-SRIPs and the luciferase activity thereof was measured.

The results of D1-SRIPs are shown in Fig. 2A. Antigen dilution rate-dependent luciferase activity was detected in all cells. This suggests that SRIPs can infect K562, Mylc, and Vero cells and replicate intracellularly. In addition, the luciferase activity of K562 cells was compared between 24 hr culture and 48 hr culture. As a result, luciferase activity was sufficiently detected even after 24 hr of culture, but shorter culture time clearly reduced luciferase activity.

Similarly, the results of D2-SRIPs, D3-SRIPs, and D4-SRIPs are shown in Fig. 2B. Luciferase activity was detected in an antigen concentration-dependent manner in any SRIPs.

### (Example 3) Functional antibody test using D1-SRIPs (Vero cell)

Vero cells were passaged in a 96-well microplate (0.25×10⁵ cells per well). The next day, 75 µL of 10 to 10240-fold step-diluted dengue antibody-positive serum (2 samples: #1 and #2) was mixed with 75 µL of an equal volume of 100-fold diluted D1-SRIPs, reacted at 37°C for 2 hr, and 40 µL of the serum-SRIP mixture was inoculated into Vero cells (Fcγ receptor-non-expressing cells) that were passaged the previous day and allowed to adsorb for additional 2 hr at 37°C. Thereafter, 100 µL of warmed 10% FBS-added DMEM was dispensed into each well, and intracellular luciferase activity was measured after 48 hr of culture at 37°C.

The results are shown in Fig. 3. Luciferase activity was suppressed (i.e., infection by SRIPs was neutralized) under conditions of low serum dilution rate (= high antibody concentration). As serum dilution rate increased, the luciferase activity approached the value of serum-free Control. When the neutralization activity of #1 and #2 was compared, luciferase activity started to increase at 1:160 for #1, whereas #2 showed sufficiently low luciferase activity even under 1:320 serum dilution conditions, suggesting that #2 has higher neutralization activity. This indicates that the neutralization activity of antibodies can be examined by using SRIPs under conditions of low serum dilution rate or relatively high antibody concentration.

### (Example 4) Functional antibody test using D1-SRIPs (K562 cells)

As K562 cells, only quasi-adherent cells that adhered to the bottom of the culture flasks were selectively passaged and used in this test. First, 36 µL of diluted (1:500) dengue antibody-positive serum (#1) and 36 µL of an equal volume of D1-SRIP (1:10 to 1:1000 dilution) were mixed in a 96-well poly-L lysine-coated microplate and reacted for 2 hr at 37°C, and then mixed with 1×10⁴ cells, 5×10⁴ cells, or 1x10⁵ cells of K562 cells (Fcγ receptor-expressing cells) (50 µL). After 24 hr of culture at 37°C, intracellular luciferase activity was measured. The same experiment was performed under serum-free conditions and used as Control.

The results are shown in Fig. 4A. As compared with the value of serum-free Control, increased luciferase activity was observed as an ADE action in the group to which diluted serum was added. Luciferase activity increased in proportion to the number of cells. SRIP antigen concentration was also positively correlated with luciferase activity. Furthermore, in order to evaluate ADE activity with objective numerical values, Fold enhancement was determined (Fig. 4B). Fold enhancement was determined by subtracting the luciferase activity of the serum-free Control from the luciferase activity (index) of the serum-added sample based on the data in Fig. 4A. Fold enhancement suggested an inversely proportional tendency with SRIP antigen concentration.

### (Example 5) Functional antibody test using D1-SRIPs (Mylc cell)

In the same manner as above, 36 µL of diluted (1:500) dengue antibody-positive serum (#1) and 36 µL of an equal volume of dengue type 1-SRIP (1:10 to 1:1000 dilution) were mixed in a 96-well poly-L lysine-coated microplate and reacted for 2 hr at 37°C, and then mixed with 1×10⁴ cells, 5×10⁴ cells, or 1×10⁵ cells of Mylc cells (Fcγ receptor-expressing cells) (50 µL). After 24 hr of culture at 37°C, intracellular luciferase activity was measured. The same experiment was performed under serum-free conditions and used as Control.

The results are shown in Fig. 5A. As with the K562 cells, luciferase activity increased in the group to which diluted serum was added as compared with the value of serum-free Control. Different from K562 cells, in Mylc cells, luciferase activity decreased inversely proportional to the number of cells. On the other hand, the SRIP antigen concentration was positively correlated with luciferase activity. Furthermore, Fold enhancement was determined and an inversely proportional tendency with SRIP antigen concentration was suggested (Fig. 5B).

The above results indicate that the combination of Fcγ receptor-expressing cells (K562 and Mylc cells) and SRIPs can be used to measure ADE activity of dengue antibody in the serum. From the data of Fold enhancement (Fig. 4B and Fig. 5B), K562 cells showed about 10000-fold enhancement at maximum, while Mylc cells about 1000-fold enhancement at maximum. In comparison between K562 cells and Mylc cells, K562 cells showed higher Fold enhancement. Fold enhancement suggested an inversely proportional relationship with SRIP antigen concentration.

### (Example 6) ADE activity measurement test using D2-SRIPs, D3-SRIPs, and D4-SRIPs

36 µL of diluted (1:100) dengue antibody-positive serum (#1) and 36 µL of an equal volume of D2-SRIPs, D3-SRIPs, or D4-SRIPs (each 1:100 dilution) were mixed in a 96-well poly-L lysine-coated microplate and reacted for 2 hr at 37°C, and then mixed with 1.5×10³ to 4.8×10⁴ K562 or Mylc cells (Fcγ receptor-expressing cells) (50 µL). After 24 hr of culture at 37°C, intracellular luciferase activity was measured.

ADE activity of dengue antibody-positive serum (#1) against SRIPs of each serotype was shown by Fold enhancement (Fig. 6). Similar to the results of ADE activity using D1-SRIPs, both K562 cells and Mylc cells stably showed high Fold enhancement (1000- to 100000-fold). The number of cells did not have a remarkable effect on Fold enhancement under the conditions of this assay.

### (Example 7) Experiment to shorten culture time (K562 cells and Mylc cells)

Dengue antibody-positive serum (#1) was fixed to a 1:500 antibody dilution rate, D1-SRIPs antigen dilution rate was set to 1:100 or 1:1000, and ADE test using K562 or Mylc cells was performed with shorter incubation times (30 min to 16 h). Specifically, 36 µL of diluted (1:500) dengue antibody-positive serum (#1) and 36 µL of each equal volume of D1-SRIPs (1:10 or 1:1000 dilution) were mixed in a 96-well poly-L lysine-coated microplate and reacted for 2 hr at 37°C, and then mixed with 1.3×10⁴ cells or 4.0×10⁴ cells of K562 or Mylc cells (Fcγ receptor-expressing cells) (50 µL). After 30 min, 1 hr, 2 hr, 5 hr, 7 hr, or 16 hr of culture at 37°C, intracellular luciferase activity was measured.

Fig. 7A shows the luciferase activity, and Fig. 7B shows the Fold enhancement calculated therefrom. As the culture time becomes shorter, the luciferase activity of the sample (#1) decreases, and all of the culture times shorter than 2 hr (2h, 1h, 30 min) had low values (less than Logic 3) of the same level as the antibody (-) Control (Fig. 7A). The luciferase activity of Control was already less than Logic 3 at 16 hr. As luciferase activity decreased, Fold enhancement also decreased, and it seemed to be less than "Logic 1=10-fold" when the culture time was less than 5 hr (Fig. 7B). In addition, lowering the antigen concentration had a large effect on luciferase activity, but the effect on Fold enhancement was relatively small. This suggests that Fold enhancement can be used as an evaluation index without being greatly influenced by antigen concentration.

### (Example 8) Investigation of optimal conditions for short-time culture-enhancement test using high concentrations of SRIP antigen

Dengue antibody-positive serum (#1) was fixed to a 1:500 antibody dilution rate, D1-SRIPs antigen dilution rate was set to 1:2 and 1:10, and ADE test was performed using Mylc and K562 cells by 5 hr culture. Specifically, 36 µL of diluted (1:500) dengue antibody-positive serum (#1) and 36 µL of equal volume of D1-SRIPs (1:2 or 1:10 dilution) were mixed in a 96-well poly-L lysine-coated microplate and reacted for 2 hr at 37°C, and then mixed with 6.9×10¹ to 1.5×10⁵ Mylc cells or K562 (Fcγ receptor-expressing cells) (50 µL). After 5 hr of culture at 37°C, intracellular luciferase activity was measured.

Fig. 8 shows Fold enhancement obtained from luciferase activity in each cell after culture for 5 hr. SRIP antigen concentration and Fold enhancement were inversely related. Furthermore, it was suggested that ADE activity could be measured in a wider range of the number of cells in Mylc cells than in K562 cells (peak at 5.6×10³ cells). On the other hand, Fold enhancement tended to increase in proportion to the number of cells in K562 cells. These results suggest that Mylc cells can be assayed under lower cell number conditions than K562 cells.

### (Example 9) Investigation of optimal conditions for long-time culture-enhancement test using low concentrations of SRIP antigen

Dengue antibody-positive serum (#1) was fixed to a 1:500 antibody dilution rate, D1-SRIPs antigen dilution rate was set to 1:100 and 1:1000, and ADE test was performed using Mylc and K562 cells by 48 hr culture. Specifically, 36 µL of diluted (1:500) dengue antibody-positive serum (#1) and 36 µL of equal volume of D1-SRIPs (1:10 or 1:1000 dilution) were mixed in a 96-well poly-L lysine-coated microplate and reacted for 2 hr at 37°C, and then mixed with 6.9×10¹ to 1.5×10⁵ Mylc cells or K562 (Fcγ receptor-expressing cells) (50 µL). After 48 hr of culture at 37°C, intracellular luciferase activity was measured.

Fig. 9 shows Fold enhancement obtained from luciferase activity in each cell after culture for 48 hr. It was suggested that too many cells at the time of infection result in lower fold enhancement in Mylc cells (peak at 5.6×10³ cells). On the other hand, Fold enhancement tended to increase in proportion to the number of cells in K562 cells (when D1-SRIPs were used at 1:1000 dilution). From the above, in order to obtain high Fold enhancement, the optimal conditions for the enhancement test are suggested to be that cells are prepared to be "5×10³ cells/well" when Mylc cells are used and "1×10⁵ cells/well" when K562 cells are used.

### (Example 10) Measurement of concentration-dependent enhancement activity of dengue antibody-positive serum using D1-SRIPs to D4-SRIPs

Dengue antibody positive serum was step-diluted, reacted with each of D1-SRIPs to D4-SRIPs antigens, infected with Mylc and K562 cells, and ADE test was performed. The assay was performed under two different conditions below.
(i) 36 µL of dengue antibody-positive sera (#1 and #2) 4-step diluted from 1:10 and 36 µL of an equal volume of D1-SRIPs to D4-SRIPs (1:10) were mixed in a 96-well poly-L lysine-coated microplate and reacted for 2 hr at 37°C, and then mixed with 5x10³ Mylc cells and 1×10⁵ K562 cells (Fcγ receptor-expressing cells) (50 µL). After 5 hr of culture at 37°C, intracellular luciferase activity was measured.
(ii) 36 µL of dengue antibody-positive sera (#1 and #2) 4-step diluted from 1:10 and 36 µL of an equal volume of D1-SRIPs to D4-SRIPs (1:1000) were mixed in a 96-well poly-L lysine-coated microplate and reacted for 2 hr at 37°C, and then mixed with 5×10³ Mylc cells and 1×10⁵ K562 cells (Fcγ receptor-expressing cells) (50 µL). After 24 hr of culture at 37°C, intracellular luciferase activity was measured.

Fig. 10 shows Fold enhancement obtained from luciferase activity in each cell after culture for (i) 5 hr or (ii) 24 hr. Fig. 10A shows the concentration-dependent enhancement activity of dengue antibody-positive serum (#1) against SRIPs of each serotype. There was no significant difference in waveform between Mylc cells and K562 cells in the concentration dependence curve after 5 hours of culture. Mylc cells after 24 hr showed lower enhancement activity against D3-SRIPs than the waveform of K562 cells (serum dilution rate 1:10 to 1:160). Similarly, Fig. 10B shows concentration dependent enhancement activity of dengue antibody positive serum (#2) against SRIPs of each serotype. There was no significant difference in waveform between Mylc cells and K562 cells in the concentration dependence curve after 5 hr of culture. Mylc cells after 24 hr showed lower enhancement activity against D3-SRIPs than the waveform of K562 cells (serum dilution rate 1:10 to 1:160). These results suggest that when the culture time was 24 hr, the enhancement activity against some SRIPs was different between Mylc cells and K562 cells.

### [Industrial Applicability]

Using the SRIPs having YFV replicon of the present invention, antibody function can be determined in a shorter period of time than conventional, whereby rapid antibody tests can be performed. This application is based on a patent application No. 2021-089309 filed in Japan (filing date: May 27, 2021), the contents of which are incorporated in full herein.

## Claims

1. A single round infectious virus particle comprising a gene comprising a region encoding a labeled protein and a region encoding non-structural (NS) proteins 1 to 5 of the yellow fever virus genome, a capsid protein of a virus, and an outer shell protein (Envelope) of an evaluation target virus.

2. The virus particle according to claim 1, wherein the gene encoding the labeled protein can be expressed only in cells infected with the single round infectious virus particle.

3. The virus particle according to claim 1 or 2, wherein the labeled protein is nanoluciferase.

4. The virus particle according to any one of claims 1 to 3, wherein the outer shell protein of the evaluation target virus is an outer shell protein of dengue virus.

5. The virus particle according to any one of claims 1 to 4, wherein a CMV promoter is linked upstream of the region encoding a labeled protein and the region encoding non-structural (NS) proteins 1 to 5 of the yellow fever virus genome, so that the gene expression is directly controlled by the CMV promoter.

6. A method for producing the single round infectious virus particle according to any one of claims 1 to 5, comprising infecting animal cells with a vector comprising a gene with a region encoding non-structural (NS) proteins 1 to 5 of the yellow fever virus genome and a region encoding a labeled protein, a vector comprising a gene encoding a capsid protein of a virus gene, and a vector comprising a gene encoding an outer shell protein of an evaluation target virus, and collecting the single round infectious virus particle according to any one of claims 1 to 5 produced within the infected cells.

7. The production method according to claim 6, wherein the gene encoding the capsid protein of the virus, and/or the gene encoding the outer shell protein of the evaluation target virus are/is linked to a CAG promoter so that the gene(s) are/is directly controlled by the CAG promoter.

8. A method for determining neutralization activity of an antibody, comprising
contacting an Fcγ receptor-non-expressing cell with the single round infectious virus particle according to any one of claims 1 to 5 in the presence of a test antibody,
culturing the cells for 24 to 48 hr, and
measuring a label in a culture medium resulting from the culture, wherein
when the level of measured label is lower than that measured in a negative control cell, the test antibody is determined to have neutralization activity on the evaluation target virus.

9. The method according to claim 8, wherein the Fcγ receptor-non-expressing cell is Vero cell.

10. A method for determining antibody-dependent enhancement ability of an antibody, comprising
contacting an Fcγ receptor-expressing cell with the single round infectious virus particle according to any one of claims 1 to 5 in the presence of a test antibody,
culturing the cells for 30 min to 24 hr, and
measuring a label in the culture medium, wherein
when the level of the measured label is higher than that measured in a negative control cell, the test antibody is determined to have antibody-dependent enhancement ability for the evaluation target virus.

11. The method according to claim 10, wherein the Fcγ receptor-expressing cell is K562 cell or Mylc cell.

12. The method according to claim 10 or 11, wherein the Fcγ receptor-expressing cell is cultured for 2 to 16 hr.

13. The method according to any one of claims 10 to 12, wherein a dilution rate of the single round infectious virus particle is 2 times or more.

14. The method according to any one of claims 10 to 13, wherein the culture of the Fcγ receptor-expressing cell is performed using Fcγ receptor-expressing cells at not less than 2.1×10² cells/well in a 96 well plate.

15. The method according to any one of claims 10 to 14, comprising adding a composition comprising the test antibody to the culture medium before contact of the single round infectious virus particle, or simultaneously with the contact of the single round infectious virus particle.

16. The method according to claim 15, wherein a mixture of the single round infectious virus particle and the composition comprising the test antibody is added to the culture medium.

17. The method according to claim 15 or 16, wherein the composition comprising the test antibody is a serum derived from a test subject.

18. The method according to claim 17, wherein the composition comprising the test antibody is a 10- to 10240-fold diluted serum derived from the test subject.

19. A composition for determining antibody function comprising the single round infectious virus particle according to any one of claims 1 to 5 as an active ingredient.

20. The composition according to claim 19, the antibody function is antibody-dependent enhancement ability or neutralization activity.

21. The composition according to claim 19 or 20 for use in the method according to any one of claims 6 to 18.

22. A kit for determining antibody function comprising the single round infectious virus particle according to any one of claims 1 to 5 as an active ingredient.

23. The kit according to claim 22, further comprising a negative control antibody or a standard antibody.

24. The kit according to claim 22 or 23, further comprising an Fcγ receptor-expressing cell.
